(19) Europäisches Patentamt / European Patent Office / Office européen des brevets

(11) **EP 2 437 667 B1**

(12) **EUROPEAN PATENT SPECIFICATION**

(45) Date of publication and mention
of the grant of the patent:
**24.03.2021 Bulletin 2021/12**

(21) Application number: **10735347.6**

(22) Date of filing: **31.05.2010**

(51) Int Cl.:
**A61B 17/00** *(2006.01)*

(86) International application number:
**PCT/IT2010/000241**

(87) International publication number:
**WO 2010/140179 (09.12.2010 Gazette 2010/49)**

(54) **A DUAL LUMEN CATHETER FOR THE INFUSION OF A BICOMPONENT HAEMOSTATIC AGENT**

DOPPELLUMENKATHETER ZUR INFUSION EINES AUS ZWEI KOMPONENTEN BESTEHENDEN HÄMOSTASEMITTELS

CATHÉTER À DEUX LUMIÈRES POUR PERFUSER UN AGENT HÉMOSTATIQUE À DEUX COMPOSANTS

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **03.06.2009 IT MI20090969**

(43) Date of publication of application:
**11.04.2012 Bulletin 2012/15**

(73) Proprietor: **Betaglue Technologies S.P.A.
20121 Milano (IT)**

(72) Inventor: **AZZI, Carlo
I-80122 Napoli (IT)**

(74) Representative: **Cooley (UK) LLP
Dashwood
69 Old Broad Street
London EC2M 1QS (GB)**

(56) References cited:
**WO-A-03/039375      WO-A-2008/151456
CA-A1- 2 636 980     FR-A- 2 722 104
US-A- 4 932 942      US-A- 5 322 510**

## Description

Field of the Invention

The present invention concerns a dual lumen catheter for the infusion of a bicomponent haemostatic agent.

Background of the Invention

Bleeding is a problem that must be faced in any surgical or para- surgical operation, as e.g. after biopsies or invasive percutaneous treatments. The control of bleeding is particularly demanding if it involves parenchymatous vascular organs like the liver or the spleen, if the origin of bleeding is difficult to reach as in the surgical treatment of a slipped disc or in laparoscopic surgery, or if the coagulative parameters of the patient have been altered by drugs like aspirin or heparin, or due to the effect of a cardiopulmonary by-pass or congenital or acquired coagulopathies .

The best local haemostatic compounds contains thrombin and fibrin glue which act onto the natural coagulative mechanism at a level different from the one of the platelet activation, and which work even better in those conditions in which the platelet activity appears to be damaged.

Also Tissucol® belongs to this class of haemostatic agents. It is a tissue adhesive containing fibrinogen, thrombin, Factor XIII and calcium chloride in order to recreate the end phases of the physiological blood coagulation, as well as the anti-fibrinolytic agent aprotinin, which inhibits the degradation of the coagulum. Tissucol® is used locally as a support treatment when standard surgical techniques are not sufficient to obtain a tissue adhesion, as a support to suture and haemostasis, as it has bilostatic and sealing properties.

All these techniques or aids may be used only in open surgery, while for local and regional mini-invasive percutaneous or laparoscopic treatments specific catheters are needed for their use and functioning.

In the medical, clinical, radiological and surgical field, different kinds of catheters are known to be used for these requirements, but they show several disadvantages.

Tissucol®, as well as other similar haemostatic agents, comprises at least two interacting solutions for obtaining the above described effects.

Said solutions must be kept separately and mixed at the appropriate moment in order to activate the haemostatic agent.

The known technique includes the use of double body and double plunger syringes, each one containing one solution per body.

Document WO 03/039375 A2 discloses a laproscopic device for the application of multi component materials to inner surgery incisions, comprising an interface coupling to a multi component materials dispenser, an elongated body having at least two inner lumens, generically proportional to the density of the components and in communication with said dispenser, and an applicator nozzle in communication with the inner lumen of said elongated body.

Dual lumen catheters are known for being connected to said syringes, comprising two separate pipes for the two solutions.

Due to the different density and viscosity of the two solutions, it often occurs that one flows into the catheter in a quantity greater than the other, and this causes the following problems:

- waste of haemostatic agent;
- it is difficult for the operator to apply the same due to the force he must apply to the syringe with both hands;
- wrong application of the haemostatic agent due to the delocalization of the end portion of the catheter, as the operator is not able to use at least one hand for keeping said catheter in place during the infusion of the haemostatic agent; said delocalization forces the operator to use a greater quantity of haemostatic agent in order to be sure that it has reached the bleeding part.

Besides, the reduced capacity of one of the two compounds favours the activation of the compound close to the end portion of the catheter, i.e. near the outlet of the two substances, which causes the obstruction of said catheter and causes the operator to apply more strength in order to overcome the resistance of the infusion of the two solutions. Moreover, the greater the strength required by the operator, the more difficult it is to keep the end portion of the catheter near the bleeding tissues in place; this implies the need for constant monitoring of the correct placing of the catheter and its possible replacing by ultrasound scan or by x-rays.

Due to said problems, until now Tissucol® has been used only for intra-surgery, while there have always been great difficulties in applications in percutaneous or laparoscopic treatments, since dual lumen catheters have been used, which were realized and destined to other purposes and which are not able to bring the two solutions simultaneously to the concerned area, with an evident waste of medicines and poor therapeutic results.

Therefore, the technical problem consists in assuring the same capacity to the two solutions which have a different viscosity, which means that they must flow and come out at the same time from the distal end or outlet of the catheter.

Summary of the Invention

Aim of the present invention is to realize a dual lumen catheter for the infusion of a bicomponent haemostatic agent, arranged for solving the above mentioned problem.

The set aim is reached by means of a dual lumen catheter for the infusion of a bicomponent haemostatic agent according to the present invention, particularly for haemostatic agents comprising thrombin and fibrinogen, according to the main independent claim 1. Further features of the invention are contained in the dependent claims.

According to a further aspect of the disclosure, it is preferred for said ratio of said areas to be greater than the square root of the ratio of the viscosities of the solutions flowing through the respective lumens.

Particularly, it is preferred for said ratio of said areas to be about equal to the square of the ratio of the viscosities of the solutions flowing through the respective lumens.

Said ratio value takes a further element into account, i.e. that the viscosity varies according to the temperature.

In a further variant said lumens have a circular cross-section and are concentric, and the first lumen incorporates the second one. In this case it is preferred for the first lumen to have such a dimension as to receive the solution with a greater viscosity, so that said solution might be exposed to a greater extent to warming due to the contact of the catheter with the patient's tissues during infusion.

Therefore, the variant of the catheter allowing to better exploit the penetration of the warmth coming from outside implies for the two lumens to have a circular cross-section and to be concentric and for the solution with a greater viscosity containing fibrinogen to flow in the most external lumen, and therefore the most external lumen to have a greater cross-section than the internal one.

The catheter according to the present invention, is applied in medical-surgical field in mini-invasive percutaneous procedures, in surgical, laparoscopic or surgical radiology treatments whenever it may be required to perform biopsies, drainages, to implant prothesis from the outside, to repair bile, pancreatic, pulmonary fistulae, after ablation treatments like: liver, pulmonary, pancreatic, mammary, renal, suprarenal, prostatic ablation etc., by means of radiofrequency, cryotherapy, laser, microwave, alcoholization, irreversible electrophoresis etc., and in all cases in which it is required to prevent or stop bleedings or haemorrhages.

Due to the present invention it becomes possible to use Tissucol® and similar for preventive and therapeutic purposes in all percutaneous surgical and/or surgical radiology procedures at low costs and with the best results, reducing the risks connected to said mini-invasive procedures to a minimum.

Said catheter may be supplied in a kit with an introducer and, if necessary, with a needle.

The dependent claims describe preferred embodiments of the invention being integral part of the present description.

Brief Description of the Figures

Further features and advantages of the invention will become more evident in the detailed description of preferred but not exclusive embodiments of a dual lumen catheter for the infusion of a bicomponent haemostatic agent, shown by way of non-limiting example, according to the enclosed drawings in which:

Figure 1 shows a cross-section of a dual lumen catheter with a circular section according to the present invention, wherein the lumens have a circular cross-section and are concentric and the external lumen has a greater area connected to its own section.

Figure 2 shows a cross-section of a dual lumen catheter with a circular section, the two lumens with circular cross-section being not concentric.

Figures 3 and 4 show cross-sections of dual lumen catheters with circular section according to the present invention, wherein the lumens are divided by a septum respectively straight and curved.

Figure 5 shows a longitudinal section of a catheter according to the preceding figures.

Figure 6 shows a longitudinal section of an introducer compatible with the catheter shown in the previous figures.

The same reference numbers and letters of the drawings identify the same elements or components.

Detailed description of a preferential embodiment

According to a preferred embodiment, a dual lumen catheter 1 according to the present invention comprises a first lumen 2 and a second lumen 3 with circular cross-section, wherein said first lumen incorporates said second lumen in order for the respective sections to be concentric.

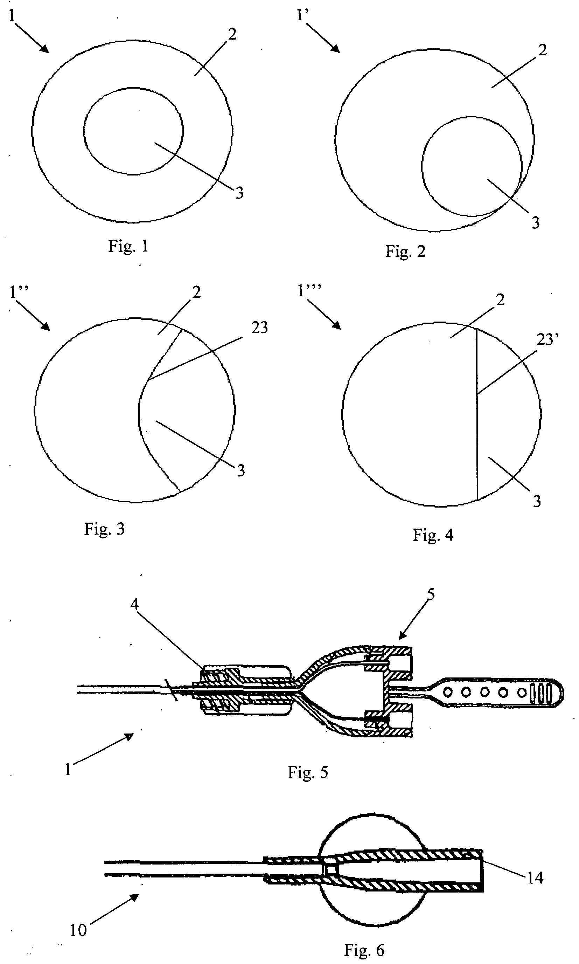

As an alternative, said two lumens are divided by a curved septum 23 or by a straight septum 23', as shown in figures 3 and 4.

The ratio between the sections of the two lumens is the result of a number of laboratory tests which have allowed to gauge the lumens according to the density and viscosity of the single components of Tissucol®, and in consideration of their reaction to temperature variations.

Such preferred ratio between the sections is about equal to the square of the ratio of the respective viscosities.

Therefore, if said first lumen is intended for the infusion of a first solution having a kinematic viscosity, expressed in the square of the length divided the time $[L^2/t]$, which is about twice as much compared to a second solution flowing in said second lumen, this means that the area of the section of the first lumen is about four times the area of the section of the second lumen.

A table is listed below showing the kinematic

viscosity expressed in mm$^2$/s of a solution containing fibrinogen at different temperatures and for different commercial products similar to Tissucol®, comprising Tissucol® itself.

| LOT | 18° C | 25° C | 37° C |
|---|---|---|---|
| 1 | 560.0 | 184.5 | 57.4 |
| 2 | 635.0 | 227.4 | 51.3 |
| 3 | 507.3 | 230.3 | 72.6 |
| 4 | 261.4 | 230.2 | 50.0 |
| 5 | 293.1 | 114.7 | 48.4 |

**viscosity expressed in mm$^2$/s**

As a matter of fact, fibrinogen is the haemostatic agent component with the greater viscosity and therefore the one causing greater problems during its perfusion. The viscosity of the solution containing fibrinogen is about double compared to the viscosity of the substance containing thrombin.

The table shows that when the temperature increases the viscosity decreases; furthermore, at the same temperature the viscosity may double (or halve) according to the commercial product or according to its production lot.

Thanks to the present invention it is possible to assure the same capacity of the two solutions, moving contemporarily along the catheter, i.e. coming out of the same in the same quantity, so as to assure a correct activation of the agent when it is infused near the bleeding tissues of a patient. As an advantage, it is possible to reduce to a minimum the perfusion times and the effort of the operator while performing the infusion; at the same time, it is possible to reduce the injected quantities of haemostatic agent to a minimum.

The features of a catheter according to the present invention are absolutely surprising: since someone skilled in the art would have clearly applied Poiseuille's Law, which states that the motion at constant speed of a viscous and uncontainable solution in a pipe having a constant section is laminar, i.e. it consists in the relative sliding of an endless number of cylinders coaxial to the axis of the pipe.

As a consequence, a pressure variation $\Delta p$ between two points respectively set at the inlet and at the outlet of the pipe is given by:

$$\Delta p = \frac{8\eta L}{\pi \cdot r^4} Q_v$$

L is the length of the pipe, r is the diameter of the pipe, $Q_v$ is the flow rate of the viscous solution in the pipe and $\eta$ is the viscosity of the solution.

Since it is required for the two solutions to have the same flow rate and the same pressure variation along the catheter, so as to minimize the effort of the operator acting on the double-body syringe; therefore:

$$\frac{\eta_1}{\pi \cdot r_1^4} = \frac{\eta_2}{\pi \cdot r_2^4},$$

that is

$$\frac{\eta_1}{S_1^2} = \frac{\eta_2}{S_2^2},$$

so

$$\frac{\eta_1}{\eta_2} = \frac{S_1^2}{S_2^2}$$

$S_1$ and $S_2$ respectively show the areas of the lumens' sections.

In other words, if Poiseuille's Law is applied, the result should be for the ratio of the sections of the two lumens to be proportional to the square root of the ratio of the respective viscosities.

To be more precise, the present invention provides for a preferred portion between ratios of the areas and ratios of the sections, expressed with the following formula:

$$\frac{\eta_1^2}{\eta_2^2} = \frac{S_1}{S_2}$$

which seems to go against said physical law.

In fact, said physical law is not able to estimate the behaviour of the two solutions composing the haemostatic agent when the temperatures vary, and particularly when the catheter is inserted in the patient's body. In fact, under said circumstances the temperature of the two solutions varies from a room temperature of about 18°C to about 37°C of the human body.

Above described formula may be varied in order to envisage different placing of the internal lumen with respect to the external one, e.g. with the lumens' axis' being parallel but not coinciding (figure 2), or with the two lumens divided by a septum (figures 3 and 4).

However, the best properties of the catheter are obtained according to the following relation:

$$\sqrt{\frac{\eta_1}{\eta_2}} < \frac{S_1}{S_2}$$

i.e. when the ratio between the areas of the lumens' sections is slightly bigger than the square root of the ratio of the respective viscosities.

One embodiment according to the present invention has a standard external diameter of 16G (Gauge), corresponding to about 1,6 mm external diameter. Furthermore, the two lumens are concentric, therefore:

- the first lumen has a diameter of 0,022 inches, i.e. 0,56 mm
- the second lumen has a diameter of 0,010 inches, i.e. 0,25 mm.

This means that the area of the section of the second lumen is $\pi \cdot 0,125^2 = 0,04906$ mm$^2$, the gross area relative to the first lumen is $\pi \cdot 0,28^2 = 0,24617$ mm$^2$ while the net area is $0,24617 - 0,04906 = 0,19711$ mm$^2$. This means that the ratio between the areas of the first and the second lumen is $\cong 4$ and fits to solutions having $a \cong \sqrt{4} = 2$ relationship between the viscosities.

Externally, said catheter may have a standardized section, e.g. 20G, 18G, 16G, 14G where G means Gauge .

Preferably, the catheter is made of a radiopaque material in order to be seen during radiography. A length compatible with its use is of about 20 cm, and it is preferred for said material to make it semi-rigid.

Preferably, said catheter comprises a rigid connector arranged for being connected to the specially provided syringes for injecting a bicomponent haemostatic agent. It may therefore comprise a rigid Y shaped connector, specifically for the two components of Tissucol® - i. e. mainly thrombin and fibrinogen - with a luer-lock kind end connection, which is a standardized connection.

Finally, it is preferred for the catheter's body to have notches at a determined distance, e.g. one centimetre one from the other, i.e. divided into centimetres for at least a portion of the catheter.

The catheter may comprise a specially provided introducer having a length equal to the one of the catheter - e.g. 20 cm - and a section compatible with one of the possible sections of the catheter. Said introducer may be divided into centimetres as well, i.e. having notches at a predetermined distance, and made of radiopaque material. Furthermore, it may have an extractable steel heart with a cutting point somewhat longer than the introducer, e.g. 210 mm long, and may comprise a luer-lock connection.

The preferred material for the catheter's body is Grilflex® ELG 6260 (PEBA). The catheter ends up with a cut orthogonal to the axis.

Figure 5 shows a longitudinal section of the catheter and particularly a male luer-lock connector 4 compatible with a female luer-lock connector 14 of the introducer 10.

Without leaving the field of the present invention, said catheter may be in a kit comprising a steel needle and an introducer. Said needle comprises an oblique, sharp-edged point and is preferably visible in ultrasound scanning.

One section of the needle could have, e.g. 16G, corresponding to 1,60 mm external diameter and 1,20 mm internal diameter in AISI 304 with the point visible in ultrasound scanning.

At the opposite end of said oblique point, said needle may have a luer-lock connector, e.g. in transparent ABS Terlux® TR2812.

The preferred length of the needle is 200 mm.

Onto said needle a plastic introducer of Grilamid® L25 may be applied, preferably with a tapered point. The internal diameter of the introducer's section is about 1,70 mm, while the length of the introducer should be preferably slightly longer than the needle, e.g. 210 mm.

It is preferable to mount a luer-lock to the introducer onto which a threaded male connector is glued, which allows to replace the steel needle with the dual lumen catheter arranged for being screwed into the introducer.

The introducer pipe is divided in centimetres and radiopaque, loaded with 30% barium sulphate and inserted in a polythene protection pipe.

Before a mini-invasive treatment takes place, the kit is arranged so that the introducer is inserted first, with the diameter corresponding to the needle or electrode needle chosen according to the procedure to be followed, and it will be placed under the guide of ultrasound scanning, CAT or MRI; once the heart of the introducer is removed, the device required for the mini-invasive procedure is inserted, like a needle, an electrode-needle, a laser fibre etc. When the treatment is ended, the device is removed and the coaxial dual lumen catheter is inserted and Tissucol® is supplied according to the needs, in order to fill the treatment area and the distance covered by the introducer, which is then slowly removed in order to release Tissucol® on its course while it is being removed.

Advantageously, the catheter may be used after any mini-invasive percutaneous procedure or during laparoscopy, after ablation treatments like radiofrequency, cryotherapy, microwave, laser, irreversible electrophoresis, needle biopsies for micro-histologicals, etc., to prevent haemorrages, bile, bronchial, pancreatic lesions and lesions from neoplastic seeding; after the emptying of dual lumens, after abscesses and complicated cysts; in the treatment of bronchial, bile and pancreatic fistulae. This assures optimal use, an easy supply, saving of the drugs and preventing further invasive manoeuvres for introducing the haemostatic agent from outside.

The described elements and features, in their different preferred embodiments, may be combined without departing from the protection scope of the present application. The invention is defined solely by the following claims.

**Claims**

1. Dual lumen catheter for the infusion of a bicomponent haemostatic agent, the dual lumen catheter comprising a first lumen (2) containing a first solution of a first component of a haemostatic agent and a second lumen (3) containing a second solution of a second component of the haemostatic agent, said lumens defining, according to a catheter cross-section, a first area and a second area respectively, **characterized in that**:

 the ratio of said areas is proportional to the viscosity ratio of the respective solutions,
 a ratio of the viscosities of the first solution to the second solution is about 2:1 from a temperature of about 18°C to about 37°C; and
 said ratio of said first area to said second area is about 4:1.

2. Catheter according to claim 1, wherein said catheter (1) comprises a circular cross-section having a standardized diameter.

3. Catheter according to claim 2, wherein said lumens are divided by a septum (23 or 23').

4. Catheter according to claim 3, wherein said septum is curved (23) or straight (23').

5. Catheter according to claim 2, wherein said lumens have a circular transversal section, said first lumen (2) incorporating said second lumen (3).

6. Catheter according to claim 5, wherein said lumens are concentric.

7. Catheter according to claim 6, wherein said section of said first lumen (2) has a diameter equal to 0.56 mm and said section of said second lumen (3) has a diameter equal to 0.25 mm.

8. Catheter according to any preceding claim, wherein said catheter (1) has a length equal to 200 mm and/or it is divided into centimetres and/or it is made of a radiopaque material and/or it includes a Y shaped connector (5) and/or a male luer-lock connector (4).

9. Dual lumen catheter according to any preceding claim, wherein said viscosity is of kinematic kind.

10. Kit for mini-invasive treatment comprising a dual lumen catheter according to any preceding claim and an introducer having a size compatible with said dual lumen catheter (1) and including a female luer-lock connector (14) compatible with said male luer-lock connector (4).

11. Kit according to claim 10, comprising a standardized steel needle including a male luer-lock connector equal to the male luer-lock connector (4) of said dual lumen catheter (1).

**Patentansprüche**

1. Doppellumenkatheter für die Infusion eines Zweikomponenten-Hämostatikums, wobei der Doppellumenkatheter Folgendes umfasst: ein erstes Lumen (2), das eine erste Lösung einer ersten Komponente eines Hämostatikums enthält, und ein zweites Lumen (3), das eine zweite Lösung einer zweiten Komponente des Hämostatikums enthält, wobei die Lumen entsprechend einem Katheterquerschnitt eine erste Fläche bzw. eine zweite Fläche definieren, **dadurch gekennzeichnet, dass**: das Verhältnis der Flächen proportional zu dem Viskositätsverhältnis der jeweiligen Lösungen ist, ein Verhältnis der Viskositäten der ersten Lösung zu der zweiten Lösung etwa 2 : 1 von einer Temperatur von etwa 18 °C bis etwa 37 °C beträgt und
 das Verhältnis der ersten Fläche zu der zweiten Fläche etwa 4 : 1 beträgt.

2. Katheter nach Anspruch 1, wobei der Katheter (1) einen kreisförmigen Querschnitt umfasst, der einen standardisierten Durchmesser aufweist.

3. Katheter nach Anspruch 2, wobei die Lumen durch ein Septum (23 oder 23') unterteilt sind.

4. Katheter nach Anspruch 3, wobei das Septum gekrümmt (23) oder gerade (23') ist.

5. Katheter nach Anspruch 2, wobei die Lumen einen kreisförmigen transversalen Abschnitt aufweisen, wobei das erste Lumen (2) das zweite Lumen (3) enthält.

6. Katheter nach Anspruch 5, wobei die Lumen konzentrisch sind.

7. Katheter nach Anspruch 6, wobei der Abschnitt des ersten Lumens (2) einen Durchmesser von gleich 0,56 mm aufweist und der Abschnitt des zweiten Lumens (3) einen Durchmesser von gleich 0,25 mm aufweist.

8. Katheter nach einem vorstehenden Anspruch, wobei der Katheter (1) eine Länge von gleich 200 mm aufweist und/oder er in Zentimeter unterteilt ist und/oder er aus einem röntgenopaken Material hergestellt ist und/oder er einen Y-förmigen Anschluss (5) und/oder einen männlichen Luer-Lock-Anschluss (4) einschließt.

9. Doppellumenkatheter nach einem vorstehenden Anspruch, wobei die Viskosität kinematischer Art ist.

10. Kit für die minimalinvasive Behandlung, der Folgendes umfasst: einen Doppellumenkatheter nach einem vorstehenden Anspruch und ein Einführmittel, das eine Größe aufweist, die mit dem Doppellumenkatheter (1) kompatibel ist, und einen weiblichen Luer-Lock-Anschluss (14) einschließt, der mit dem männlichen Luer-Lock-Anschluss (4) kompatibel ist.

11. Kit nach Anspruch 10, der eine standardisierte Stahlnadel umfasst, die einen männlichen Luer-Lock-Anschluss einschließt, der dem männlichen Luer-Lock-Anschluss (4) des Doppellumenkatheters (1) entspricht.

## Revendications

1. Cathéter à deux lumières pour la perfusion d'un agent hémostatique à deux composants, le cathéter à deux lumières comprenant une première lumière (2) contenant une première solution d'un premier composant d'un agent hémostatique et une deuxième lumière (3) contenant une deuxième solution d'un deuxième composant de l'agent hémostatique, lesdites lumières définissant respectivement, en fonction d'une section transversale du cathéter, une première zone et une deuxième zone, **caractérisé en ce que** :

le rapport desdites zones est proportionnel au rapport des viscosités des solutions respectives,
un rapport de la viscosité de la première solution sur la viscosité de la deuxième solution est d'environ 2:1 à partir d'une température d'environ 18 °C à environ 37 °C ; et
ledit rapport de ladite première zone sur ladite deuxième zone est d'environ 4:1.

2. Cathéter selon la revendication 1, dans lequel ledit cathéter (1) comprend une section transversale circulaire ayant un diamètre standardisé.

3. Cathéter selon la revendication 2, dans lequel lesdites lumières sont divisées par un septum (23 ou 23').

4. Cathéter selon la revendication 3, dans lequel ledit septum est incurvé (23) ou rectiligne (23').

5. Cathéter selon la revendication 2, dans lequel lesdites lumières ont une section transversale circulaire, ladite première lumière (2) incorporant ladite deuxième lumière (3).

6. Cathéter selon la revendication 5, dans lequel lesdites lumières sont concentriques.

7. Cathéter selon la revendication 6, dans lequel ladite section de ladite première lumière (2) a un diamètre égal à 0,56 mm et ladite section de ladite deuxième lumière (3) a un diamètre égal à 0,25 mm.

8. Cathéter selon l'une quelconque des revendications précédentes, dans lequel ledit cathéter (1) a une longueur égale à 200 mm et/ou il est divisé en centimètres et/ou il est fait d'un matériau radio-opaque et/ou il comporte un raccord en forme de Y (5) et/ou un raccord luer lock mâle (4) .

9. Cathéter à deux lumières selon l'une quelconque des revendications précédentes, dans lequel ladite viscosité est du type cinématique.

10. Kit de traitement mini-invasif comprenant un cathéter à deux lumières selon l'une quelconque des revendications précédentes et un introducteur ayant une taille compatible avec ledit cathéter à deux lumières (1) et comportant un raccord luer lock femelle (14) compatible avec ledit raccord luer lock mâle (4).

11. Kit selon la revendication 10, comprenant une aiguille en acier standardisée comportant un raccord luer lock mâle égal au raccord luer lock mâle (4) dudit cathéter à deux lumières (1).

Fig. 1

Fig. 2

Fig. 3

Fig. 4

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03039375 A2